# EUROPEAN PATENT APPLICATION

(11) **EP 1 234 871 A1**
(43) Date of publication of application: **28.08.2002**
(21) Application number: 02251199.2
(22) Date of filing: 21.02.2002
(51) Int. Cl.: C12M 1/30

(54) **A swab assembly**

(30) Priority: 21.02.2001 GB 0104246; 07.11.2001 GB 0126679
(71) Applicant: Hayton, Anthony John, Rossendale, Lancashire BB3 4EA (GB); Kinsella, Roger James, Altrincham, Cheshire WA15 4QN (GB)
(72) Inventor: Hayton, Anthony John, Rossendale, Lancashire BB3 4EA (GB); Kinsella, Roger James, Altrincham, Cheshire WA15 4QN (GB)
(74) Representative: Wilson Gunn M'Caw

(57) **Abstract**

A swab assembly (10) comprises an external housing (11) formed from two interengageable housing parts (12, 13). A hollow tubular member (14) having swab material (16) at one end is attached to one housing part (12) at the other end. The interior of the tubular member (14) is closed off with a seal member and contains a suitable reagent (18) which is held in spaced disposition from the swab material by capillary action. When the assembly is to be used, the two housing parts (12, 13) are separated revealing the inner tubular member (14) and the swab material (16) can be utilised to collect a sample. If it is desired to dispense reagent (18) onto the swab material (16) prior to or after collection of the sample, the tubular member (14) can be deformed in the region of a seal (17) to separate the tubular member from the housing part (12) thereby allowing the reagent (18) to flow onto the material (16).

## Description

This invention relates to a swab assembly and particularly but not exclusively to a swab assembly for collection of samples or specimens for analysis.

Swab assemblies are used for collection of samples or specimens for analysis in many different fields for example in the pharmaceutical industry, food industry, chemical industry and medical and forensic fields.

In these fields it may be necessary for swab material in the assembly to be treated with a predetermined quantity of a reagent either prior to, or subsequent to, collection of the sample or specimen. Problems can arise where the swab material is treated with a reagent prior to use, since if the delay between treating and use is sufficiently long in duration, when used the swab material may separate from the assembly rendering the assembly useless. A further problem which arises is that it can be difficult to ensure that the correct quantity of reagent is used. The importance of this in some fields can be appreciated since it might be the case that if too much reagent is used, this may result in an unacceptable adverse effect in the sample or specimen such as washing away of the collected sample or specimen. If too little is used, this may result in an unacceptable analysis of the sample or specimen, or if the reagent is necessary to fix or preserve the sample or specimen, it may result in unacceptable degradation of the sample or specimen.

It is an object of the present invention to provide a swab assembly which overcomes the above mentioned problems and which is easy to use effectively.

Thus and in accordance with the present invention therefore there is provided a swab assembly comprising an outer housing and an inner hollow tubular member having swab material attached to one end thereof and a seal provided at another end, a predetermined quantity of a reagent being held in said tubular member between said swab material and said seal but spaced from said swab material by capillary action, said inner hollow tube being adapted to be frangible adjacent said seal wherein, at least a part of said external housing is formed of resiliently deformable material whereby said frangible part of said tubular member can be fractured by deformation of said part of said external housing thereby allowing said reagent to flow onto said swab material.

With this arrangement, it will be appreciated that a predetermined selected quantity of reagent can be dispensed onto a swab material either prior to, or subsequent to, the collection of a sample or specimen on the swab material. Furthermore, the provision of the swab material in an outer housing, means that prior to use there is no fear of contamination of the device and also provides a convenient housing into which the swab material can be placed subsequent to collection to prevent any further contamination.

Preferably the outer housing is found in at least two interengageable housing parts. Preferably an end of said hollow tubular member is nonreleasably secured to one said housing part for ease of handling.

Preferably said frangible part is formed in an area of reduced thickness as compared to the remainder of the tubular member.

Most preferably the swab material is formed of a textile or foamed plastics material, for example cotton, synthetic cloths. Alternatively, the swab material may comprise a sponge material or any other suitable material.

Preferably the inner tubular member is formed from a plastics material and has a diameter in the range 0.5 to 10.0 mm and most preferably in the range 0.5 to 3.0 mm.

Furthermore, the seal may comprise any suitable material capable of forming an airtight liquid proof barrier, for example wax, silicon or may comprise a heat seal or crimping in the tubular member itself.

The invention will now be described further by way of example only and with reference to the accompanying drawings in which:
**Fig. 1** shows a schematic representation of one form of swab assembly according to the invention; and
**Fig. 2** shows a schematic representation of a second form of swab assembly according to the invention.

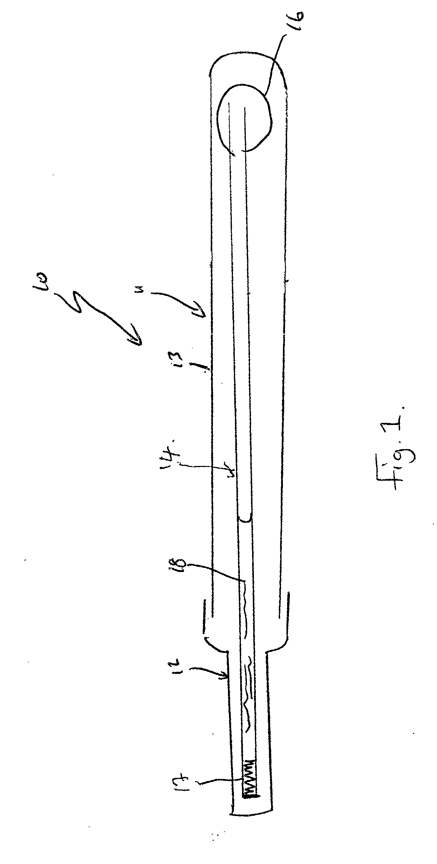

Referring now to the drawings there is shown in Fig. 1, a swab assembly 10 comprising an external housing 11 formed from two interengageable housing parts 12, 13. Each housing part 12, 13 is formed from a plastics material and at least one housing part 12 is formed from a resiliently deformable plastics material. As shown in the drawing, one part of the housing 12 is shaped so as to be a push fit on an external surface of the other housing part 13 to provide the interengagement. Alternatively the one housing part 12 may engage an internal surface of the other housing part 13 or indeed the housing parts 12, 13 may cooperate in any other suitable manner to achieve interengagement.

Internally of the external housing 11 is a hollow tubular member 14 having swab material 16 at one end. An opposite end of the member is attached to an internal surface of the housing part 12 and a hollow interior is closed off with a seal member 17. A reagent 18 of suitable form in a predetermined quantity, is contained within the interior of the hollow tubular member 14 between the seal member 17 and the swab material and is held in spaced disposition from said swab material 16 by capillary action. The hollow tubular member 14 is adapted to be frangible adjacent the seal member 17 and thus the tubular member may, in a preferred embodiment, comprise an area of weakness adjacent the seal member 17 which when distorted causes fracture of the tubular member 14 in the area of weakness.

The seal 17 may comprise any suitable material such as, for example a wax, silicon based material or may just be an area in which the tubular member has been heat sealed or crimped. The seal provides an airtight liquid barrier in order that the capillary action can take effect.

The swab material 16 may comprise any suitably absorbent material such as, for example cotton, sponge, synthetic or textile cloths.

In use, the assembly is provided as shown in the drawing. Therefore, when, for example, the assembly is being used in the forensic field at a crime scene, the person wishing to utilise the assembly to collect a sample or specimen would firstly remove the hollow tubular member 14 from the outer housing 11 by disengaging the housing part 12 from the other housing part 13 by a sliding action. As the end of the hollow tubular member 14 is fixed into the housing part 12, removal of the housing part 12 will mean that the tubular member 14 can be removed from the external housing 13. Furthermore, by arranging for the tubular member 14 to be retained by the housing part 13 means that at no point does a person using the assembly have to touch the swab material 16 therefore reducing to a minimum the risk of contamination.

Once removed from the housing part 13, the swab material 16 can be used to collect a desired sample or specimen. The tubular member 14 is then replaced in the housing part 13 and the two housing parts 12, 13 are interengaged to form the complete assembly. In this position it will be realised that the sample or specimen collected on the swab material 16 is held in a suitable protective environment where the risk of contamination is minimal.

Depending upon the field in which the assembly is being used, it might be necessary to apply a suitable reagent to the swab material 16 prior to collection of a sample or specimen. This is usually the case in the field of forensic use but may also apply in other fields of use. If this is necessary, prior to use, the housing part 12 is deformed by a user in the region of the seal 17 on the tubular member 14 to such an extent that the area of weakness in the end of the hollow tubular member 14 is fractured. As the predetermined quantity of chemical reagent contained within the hollow tubular member 14 is held spaced from the swab material 16 by capillary action, as soon as the end of the hollow tubular member 14 is fractured and air can enter, the liquid flows down the hollow tubular member 14 and onto the swab material 16. It will be realised that by use of capillary action to hold the reagent 18 spaced from the swab material 16 and then to deliver the reagent onto the swab material results in a particularly effective and controlled application of reagent to the swab material and hence the collected specimen.

Once the reagent 18 has been delivered to the swab material 16, the assembly can be used as described above.

Alternatively it may be desirable or necessary that the reagent 18 is delivered to the swab material 16 after collection of the specimen and, in these circumstances, the outer housing part 12 is deformed by a user to cause fracture of the inner hollow tubular member 14 in the region of the area of weakness subsequent to collection of the sample or specimen and once the hollow tubular part 14 has been replaced in housing part 13 and the housing parts 12, 13 have been interengaged to form the complete assembly. Alternatively, the reagent 18 may be delivered onto the sample or specimen collected on the swab material 16 before the complete assembly is formed.

The choice of reagent 18 will vary according to the field of application of the assembly, for example in the field of forensic use a fixative or moistening agent may be a suitable reagent.

It will be appreciated that with this arrangement, it is possible to provide a swab assembly which is particularly easy and simple to use effectively and in which it is possible to minimise the risk of contamination of collected specimens whilst also allowing accurate delivery of a suitable reagent to a swab material either prior to, or subsequent to, sample or specimen collection.

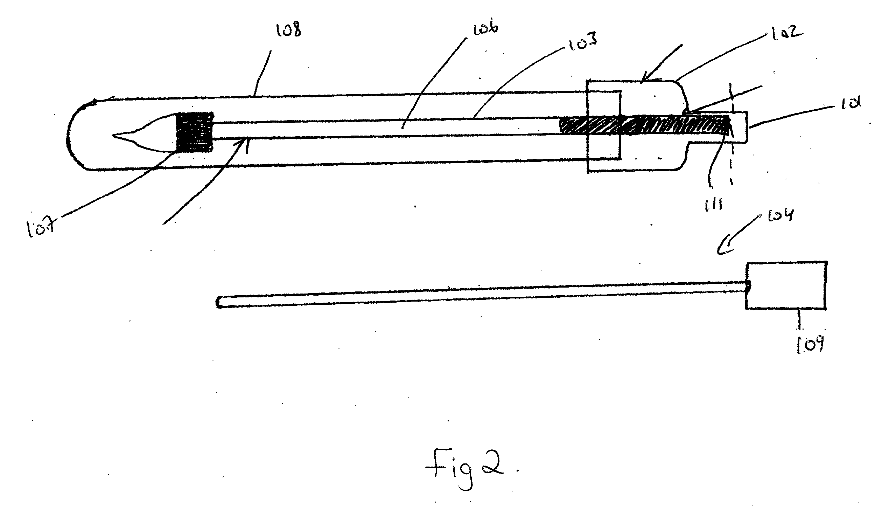

Referring now to Fig. 2, there is shown a second embodiment in which an end 101 of the housing part 102 is adapted for separation from the remainder of the housing part 102 to allow access to the hollow internal tubular member 103. This allows a plunger member 104 of appropriate dimension to be inserted into a bore 106 of the tubular member 103 and used to push the swab material 107 from the end of the member 103 so as to be retained in the other housing part 108. For this purpose the swab material 107 is adapted to be removable from the tubular member 103.

The plunger 104 has a boss part 109 which is of greater dimension than the bore 106 of the hollow tubular member 103 to prevent the plunger 104 being inserted completely into the bore 106.

In use, as disclosed in relation to the embodiment of Fig. 1 described above, liquid is released onto the swab material 107 by deforming the housing part 102 until the hollow tubular member 103 fractures adjacent the seal 111. The swab is then removed from the housing part 108, used for its purpose, and replaced in the housing part 108. The end 101 of the housing part 102 is removed, for example by cutting and the plunger member 104 is inserted in the bore 106 of the hollow tubular member 103 and is pushed in until it engages the swab material 107 and is used to disengage the material 107 from the end of the hollow member 103. The remaining part of the housing part 102 and tubular member 103 can be discarded and a cap (not shown) or similar provided to close off the open end of the housing thereby retaining the swab material in sterile conditions.

It is of course to be understood that the invention is not intended to be restricted to the details of the above embodiment which are described by way of example only.

## Claims

1. A swab assembly comprising an outer housing and an inner hollow tubular member having swab material attached to one end thereof and a seal provided at another end, a predetermined quantity of a reagent being held in said tubular member between said swab material and said seal but spaced from said swab material by capillary action, said inner hollow tube being adapted to be frangible adjacent said seal wherein, at least a part of said external housing is formed of resiliently deformable material whereby said frangible part of said tubular member can be fractured by deformation of said part of said external housing thereby allowing said reagent to flow onto said swab material.

2. A swab assembly according to Claim 1, wherein the outer housing is formed in at least two interengageable housing parts.

3. A swab assembly according to Claim 1 or Claim 2, wherein an end of the hollow tubular member is nonreleasably secured to one said housing part for ease of handling.

4. A swab assembly according to any one of Claims 1 to 3, wherein the frangible part is formed as an area of reduced thickness as compared to the remainder of the tubular member.

5. A swab assembly according to any one of Claims 1 to 4, wherein the swab material is formed of a textile or foamed plastics material.

6. A swab assembly according to Claim 5, wherein the material comprises cotton or a synthetic cloth.

7. A swab assembly according to Claim 5, wherein the material comprises a sponge material.

8. A swab assembly according to any one of Claims 1 to 7, wherein the inner tubular member is formed from a plastics material.

9. A swab assembly according to Claim 8, wherein the diameter of the inner tubular member is in the range 0.5 to 10.0 mm.

10. A swab assembly according to Claim 8, wherein the diameter of the inner tubular member is in the range of 0.5 to 3.0 mm.

11. A swab assembly according to any preceding Claim, wherein the seal comprises wax or silicon.

12. A swab assembly according to any one of Claims 1 to 10, wherein the seal comprises a heat seal or crimping in the tubular member.

13. A swab assembly substantially as hereinbefore described with reference to the accompanying drawings.
